# EUROPÄISCHE PATENTSCHRIFT

(11) **EP 1 248 578 B1**
(45) Veröffentlichungstag und Bekanntmachung des Hinweises auf die Patenterteilung: **29.10.2003**
(21) Anmeldenummer: 00916995.4
(22) Anmeldetag: 23.03.2000
(51) Int. Cl.: A61F 2/06, A61B 17/11

(54) **EINFÜHRUNGSBESTECK FÜR EINE PROTHESEN-SCHLAUCHVERBINDUNG**
INSTRUMENT FOR INSERTING A PROSTHESIS TUBE CONNECTION
INSTRUMENT DE PENETRATION POUR RACCORDEMENT TUBULAIRE PROTHETIQUE

(30) Priorität: 20.01.2000 DE 10002318
(43) Veröffentlichungstag der Anmeldung: 16.10.2002
(73) Patentinhaber: Metz, Katharina, 15741 Bestensee (DE)
(72) Erfinder: METZ, Ludwig DI, . (DE); GUSSMANN, Andreas, D-14532 Kleinmachnow (DE)
(74) Vertreter: Manitz, Finsterwald & Partner GbR
(86) Internationale Anmeldenummer: EP0002611
(87) Internationale Veröffentlichungsnummer: WO01052769

(56) Entgegenhaltungen:
- WO-A-97/43961
- WO-A-98/19607
- WO-A-98/19629

## Beschreibung

Die Erfindung betrifft ein Einführungsbesteck für eine Prothesen-Schlauchverbindung nach dem Patentanspruch 1.

Eine Prothesen-Schlauchverbindung nach Anspruch 1 ist aus der PCT-Anmeldung PCT/EP99/04192 (WO-A-0016719) bekannt. Diese Anmeldung fällt unter Art 54(3) EPÜ.

Die Erfindung nach Anspruch 1 hat ein Einführungsbesteck für die aus WO-A-0016719 schaunte Prothesen-Schlauchverbindung zum Ziel, mit denen das Metallgitter (Stent) der Prothesen-Schlauchverbindung einfach, sicher und ohne Verletzungsgefahr so in eine Arterie eingebracht und expandiert werden kann, daß anschließend ohne das Erfordernis einer Vernähung ein einwandfreier Sitz des Metallgitters in der Arterie gewährleistet ist.

Der Erfindungsgedanke ist darin zu sehen, daß man durch Heranklappen des durch das Metallgitter mit Innenverkleidung und/oder Ummantelung gebildeten T-Querbalkens an den Verbindungsschlauch und Halten in dieser Position mittels eines diesbezüglich ausgebildeten Katheters zunächst ein insgesamt längliches Gebilde schafft, welches vorzugsweise durch ein Schleusenrohr und eine zuvor durch Punktion und Dilatation hergestellte Öffnung in eine Arterie eingeführt werden kann, wo durch Aufhebung einer zuvor vorgesehenen Verankerung der Winkel zwischen Verbindungsschlauch und Metallgitter wieder vergrößert und das Metallgitter an Ort und Stelle gebracht wird. Anschließend erfolgt dann ein Expandieren des im Metallgitter vorhandenen Ballons über den Druckfluid-Zuführungsschlauch, bis das Metallgitter gegebenenfalls mit seiner Ummantelung fest an der Innenwand der Arterie anliegt und somit blutdicht mit dieser verbunden ist. Anschließend wird der Ballon über den Zuführungsschlauch druckentlastet und mit diesem durch den Verbindungsschlauch herausgezogen. Dabei wird zur Sicherung der Dilatationsöffnung im Abgangsbereich der Prothese eine erneute Aufdehnung durchgeführt. Dann kann der Verbindungsschlauch mit seinem anderen Ende blutleitend an die andere Arterie bzw. den anderen Arterienbereich angeschlossen werden, was - falls der betreffende Arterienbereich gut zugänglich ist - durch Vernähen oder ebenfalls durch ein Metallgitter mit Innenverkleidung und/oder Ummantelung erfolgen kann.

Der Katheter ist erfindungsgemäß zweckmäßigerweise nach Anspruch 2 ausgebildet.

Besonders vorteilhaft sind die Weiterbildungen nach den Ansprüchen 3 und 4, weil hierdurch die Lösung des Katheters vom Verbindungsschlauch erleichtert ist, ohne daß die Einführung des Metallgitters in die Arterie behindert ist.

Von besonderer Bedeutung ist die Ausführungsform nach Anspruch 5, weil hierdurch der T-Querbalken in Längsrichtung des Katheterrohres gehalten werden kann, bis die Einführung in die Arterie erfolgt ist.

Vorteilhafte Weiterbildungen dieser Ausführungsform entnimmt man den Patentansprüchen 6 bis 12. Die Kombination von rinnenförmigem Vorsprung und distal anschließendem Katheterrohr ermöglicht einen sicheren Halt des T-querbalkenartigen Metallgitters mit Innenverkleidung und/oder Ummantelung zumindest im wesentlichen parallel zur Katheterachse bzw. zu dem an seiner Außenseite entlanggeführten Verbindungsschlauch bis zum Einführen in die Arterie.

Weitere vorteilhafte Ausführungsformen der Erfindung, die zum Einführungsbesteck gehörende vorteilhafte Bauelemente beschreiben, sind durch die Patentansprüche 13 bis 24 gekennzeichnet.

Die Erfindung wird im folgenden beispielsweise anhand der Zeichnung beschrieben; in dieser zeigt
- Figur 1: in einer schematischen Längsachnittansicht den einen Endbereich einer Prothesen-Schlauchverbindung gemäß der älteren Anmeldung PCT/EP99/04192 (WO-A-0016719) nach Einbringung in eine Arterie und Expansion,
- Figur 2: eine teilweise geschnittene Draufsicht des Gegenstandes der Figur 1 nach Linie II-II in Figur 1,
- Figur 3: eine schematische Seitenansicht einer erfindungsgemäßen Prothesen-Schlauchverbindung im zusammengefalteten Zustand mit eingebrachtem Ballon und Druckgas-Zuführungsschlauch vor dem Aufblasen und Expandieren des Metallgitters, wobei die sie aufnehmende Arterie nicht dargestellt ist,
- Figur 4: eine etwas vergrößerte Ansicht analog Figur 3 nach dem Einbringen des ummantelten Metallgitters in eine Arterie und Aufblasen des Ballons sowie Expansion des Metallgitters,
- Figur 5: eine schematische teilweise geschnittene Seitenansicht eines Trokars mit darin angeordnetem Schleusenrohr, in dem sich ein Dilatationsrohr mit Kanüle befindet, welches in einer Arterie angeordnet gezeigt ist,
- Figur 6: eine separate Seitenansicht der innerhalb des Dilatationsrohres nach Figur 5 angeordneten Kanüle mit darin befindlichem und aus beiden Enden vorstehendem Draht,
- Figur 7: eine vergrößerte Ausschnittsansicht des proximalen Endbereiches nur der Kanüle nach Figur 5, 6 nach Punktierung einer Arterie,
- Figur 8: eine schematische Seitenansicht des Schleusenrohres nach Figur 5 in einer verkürzten Darstellung, wobei in ein Ventil am distalen Ende des Schleusenrohrs eine Einführungshülse eingebracht ist, um ein dahinter dargestelltes Katheterrohr in das Schleusenrohr problemlos einbringen zu können,
- Figur 8a: eine vergrößerte perspektivische Ansicht des proximalen Endbereiches des Schleusenrohres nach den Figuren 5 und 8,
- Figur 9: eine schematische Seitenansicht des auch in den Figuren 5 und 8 gezeigten Schleusenrohres im in eine Arterie eingebrachten Zustand mit darin angeordnetem, eine ProthesenSchlauchverbindung tragenden Katheter,
- Figur 10: einen vergrößerten Ausschnitt des proximalen Endbereiches des die Prothesen-Schlauchverbindung tragenden Katheters nach Figur 9,
- Figur 11: eine schematische Schnittansicht nach Linie XI-XI in Figur 10,
- Figur 12: eine schematische Seitenansicht einer bevorzugten Ausführungsform eines erfindungsgemäßen Ballons mit davon abzweigendem Zuleitungsschlauch im aufgeblasenen Zustand des Ballons,
- Figur 13: eine schematische Seitenansicht des Zuführungsschlauches und des Ballons im entlüfteten Zustand während des Herausziehens aus dem Verbindungsschlauch der erfindungsgemäßen Prothesen-Schlauchverbindung, welcher nicht gezeigt ist und
- Figur 14: eine vergrößerte schematische Seitenansicht eines bevorzugt erfindungsgemäß verwendeten Expansionsballons mit Druckgas-Zuführungsschlauchanschluß und Führungskanälen für den in Figur 5 und 7 dargestellten Draht.

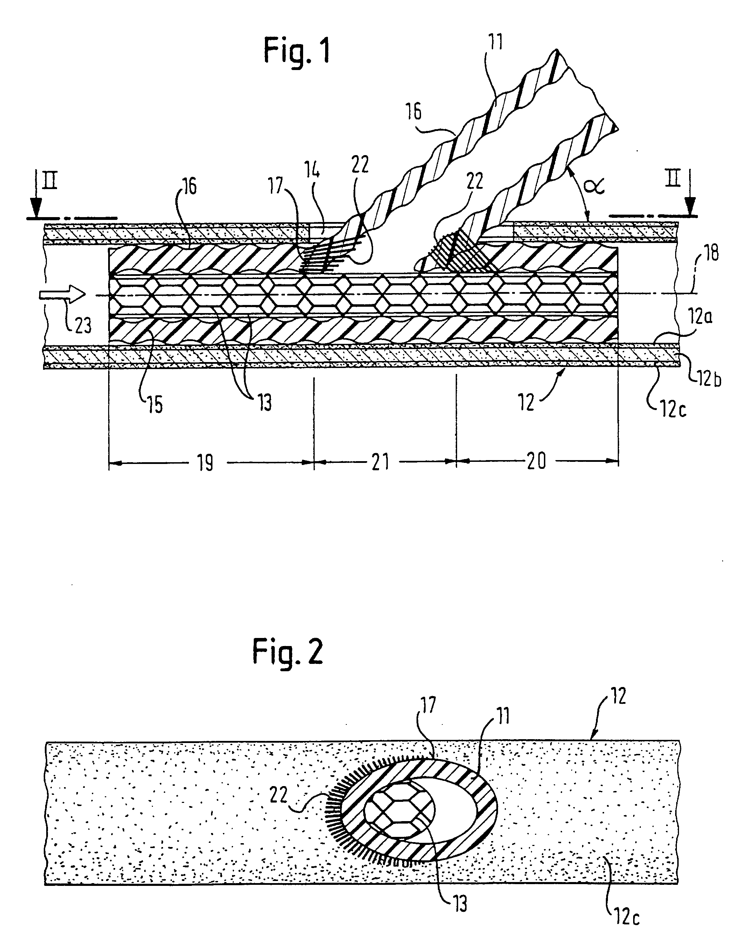

Nach Figur 1 ist ein rohrförmiges Metallgitter 13 mit einer Kunststoffummantelung 15 versehen. Derartig ummantelte Metallgitter 13 (ummantelte Stents) sind aus der Aortenchirurgie bekannt, um beispielsweise die Hauptschlagader aufzuweiten bzw. aufgeweitete Arterien oder Aortenabschnitte im Durchmesser zu reduzieren bzw. geborstene Aortenabschnitte abzudichten.

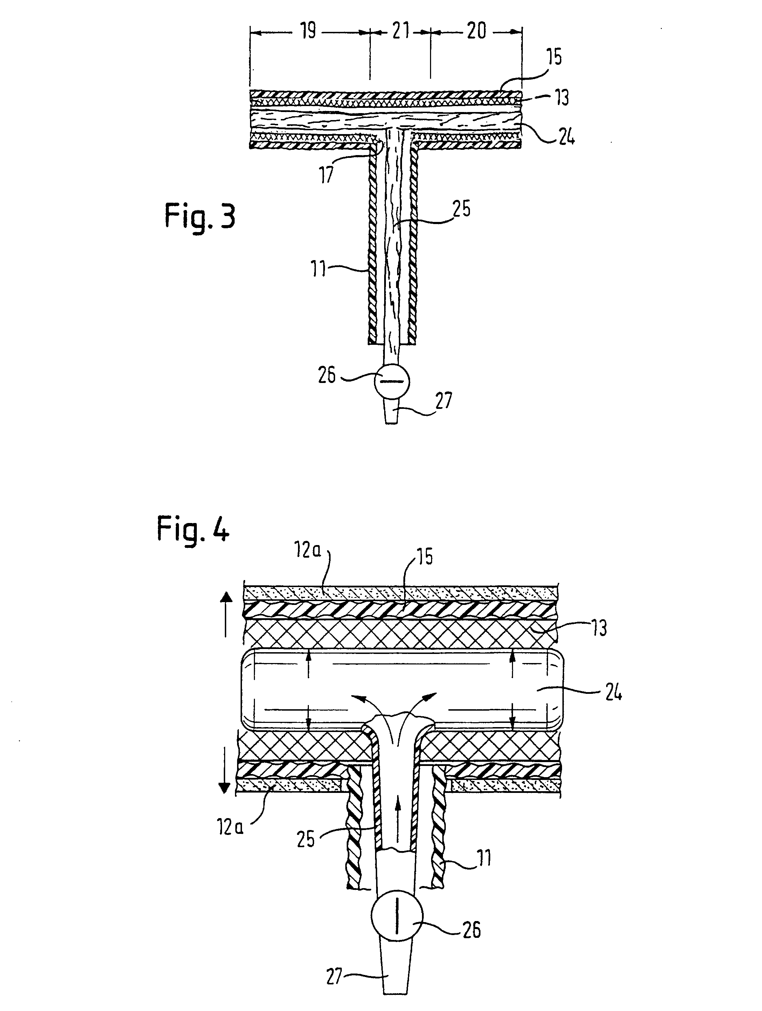

Die Ummantelung 15 ist an einer von beiden Enden entfernten Stelle auf ihrem Umfang mit einer in Richtung der Mittelachse 18 des Metallgitters 13 länglichen Öffnung 17 versehen, in die unter einem Winkel α, der nach den Figuren 3, 4 auch 90° betragen kann, das eine Ende eines Prothesen-Verbindungsschlauches 11 eingesetzt ist, und zwar derart, daß das Material des Schlauches 11 bis zur Anlage am Metallgitter 13 kommt. In diesem Zustand ist das Ende des Schlauches 11 mit den die Öffnung 17 umgebenden Bereichen der Ummantelung 15 durch eine Naht 22 blutdicht verbunden. Auf diese Weise ist an dem mit der Ummantelung 15 versehenen Metallgitter 13 ein Anschlußbereich 21 für den Verbindungsschlauch 11 gebildet, von dem aus sich das Metallgitter 13 mit der Ummantelung 15 in beiden entgegengesetzten Längs-Richtungen erstreckt. Der sich entgegen der Blutstromrichtung 23 erstreckende Teil 19 ist dabei etwas länger als der sich in Blutstromrichtung 23 erstreckende Teil 20 des Metallgitters 13 mit der Ummantelung 15.

Wie aus Figur 2 ersichtlich ist, ist die Öffnung 17 aufgrund des schrägen Anschlusses des Endbereiches des einen kreisförmigen Querschnitt aufweisenden Verbindungsschlauches 11 an die Ummantelung 15 oval ausgebildet, wobei die längere Achse der Öffnung 17 in Richtung der Mittelachse 18 des Metallgitters 13 weist.

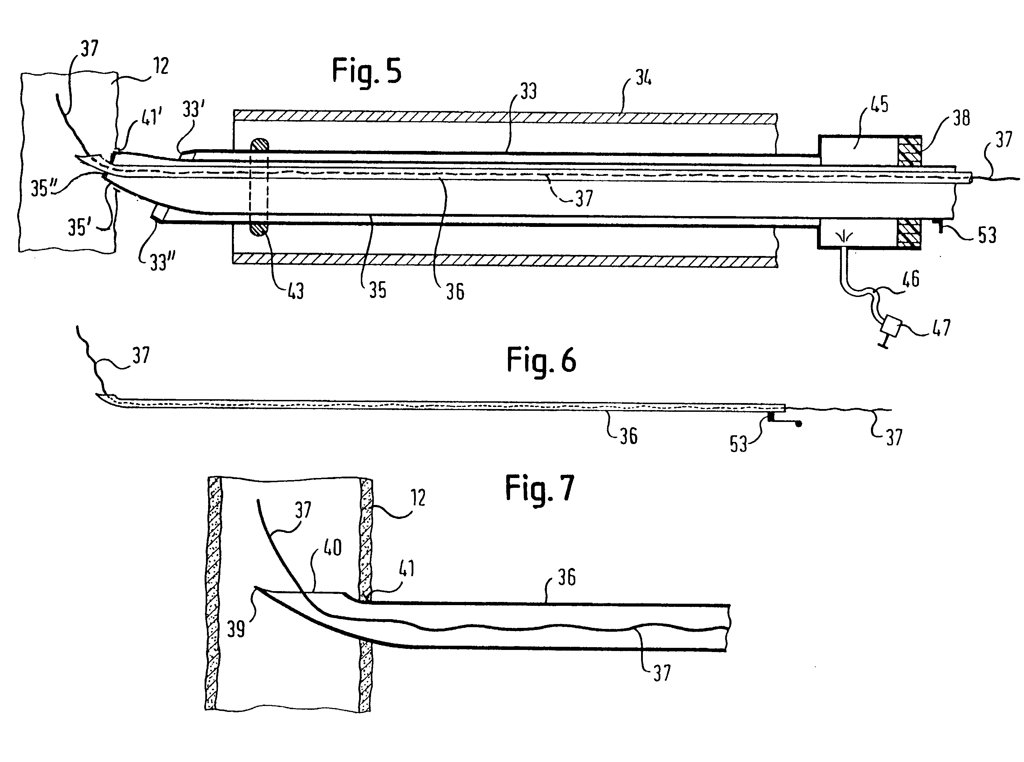

In den Figuren 1 und 2 ist die beschriebene Schlauchverbindung in einer Arterie 12 angeordnet dargestellt, welche aus drei Schichten besteht, und zwar der innenliegenden Intima 12a, der mittleren Media 12b und der außenliegenden Adventitia 12c. Eingebracht wird das Metallgitter 13 mit der Ummantelung 15 in die Arterie 12 dadurch, daß in dieser eine entsprechend der Öffnung 17 runde oder ovale Öffnung 14 vorgesehen wird. Anschließend wird dann mittels eines weiter unten anhand der Figuren 5 bis 11 beschriebenen Einführungsbestecks zunächst der längere Teil 19 in gemäß Figur 3 gefalteter Form durch die Öffnung 14 in die Arterie 12 eingeschoben, und zwar so weit, daß auch der kürzere Teil 20 gefaltet in das Innere der Arterie 12 gelangt und anschließend in die in Figur 1 dargestellte Position verschoben werden kann.

Gemäß Figur 3 ist in das gefaltete Metallgitter 13 ein sich in Richtung seiner Längsachse erstreckender länglicher Ballon 24 in entlüfteter Form eingebracht, von dem im mittleren Bereich seitlich ein Zuleitungsschlauch 25 abzweigt, der durch den Verbindungsschlauch 11 hindurchgeführt ist und in einem von Hand zu öffnenden bzw. zu schließenden Ventil 26 mündet, das einen Anschlußkonus 27 aufweist, welcher mit einer Druckquelle verbindbar ist. Durch den Zuleitungsschlauch 25 kann ein Druckgas, aber auch eine Druckflüssigkeit, z.B. eine Röntgen-Kontrastmittellösung zugeführt werden.

Im Gegensatz zum Ausführungsbeispiel nach Figur 1 zweigt der Verbindungsschlauch 11 bei der Ausführungsform nach Figur 3 und 4 senkrecht vom Metallgitter 13 mit Ummantelung 15 ab.

Nachdem der durch das Metallgitter 13 mit Ummantelung 15 gebildete T-Querbalken der Prothesen-Schlauchverbindung nach Figur 3 mit entlüftetem Ballon 24 in der weiter unten beschriebenen Weise in eine Arterie 12 eingebracht worden ist, wird der Anschlußkonus 27 an eine Druckluftquelle angeschlossen und das Ventil 26 geöffnet, worauf gemäß Figur 4 Druckluft in das Innere des Ballons 24 strömen kann, so daß der Ballon 24 sich aufbläht und eine Expansion des Metallgitters 13 mit der Ummantelung 15 so lange erfolgt, bis ein fester Sitz des Metallgitters 13 mit der Ummantelung 15 innerhalb der Arterie 12 gewährleistet ist, ohne daß es eines Vernähens bedarf.

Der Winkel α ist nach Fig. 1 so gewählt, daß das in Richtung des Pfeiles 23 strömende Blut nicht unter einem Winkel von 90°, sondern vielmehr unter einem deutlich kleineren Winkel als 90°, beispielsweise 45° vom Inneren des Metallgitters 13 in den Schlauch 11 fließen kann. Ein Teil des Blutes kann jedoch durch das Metallgitter 13 hindurch am Schlauch 11 vorbei in den hinter dem Metallgitter 13 befindlichen Teil der Arterie 12 fließen. Nach Fig. 3, 4 ist aber auch ein senkrechtes Abzweigen des Verbindungsschlauches 11 möglich.

Am in der Zeichnung nicht dargestellten anderen Ende des Prothesen-Schlauches 11 kann ebenfalls ein Metallgitter 13 mit Ummantelung 15 ähnlich wie in Figur 1 dargestellt angeordnet sein, um auch das andere Ende des Schlauches 11 in ähnlicher Weise mit einer anderen Arterie bzw. einem anderen Arterienbereich verbinden zu können, ohne daß es zur Verbindung der Herstellung einer Naht bedarf. Der Verbindungsschlauch 11 kann jedoch an dem betreffenden Ende auch mit der Arterie vernäht werden, sofern der Verbindungsbereich von außen gut zugänglich ist.

Grundsätzlich kann statt der Ummantelung 15 oder zusätzlich zu dieser auch eine Innenverkleidung am Metallgitter 13 vorgesehen sein, die in der Zeichnung nicht gezeigt ist. In diesem Fall müßte die Innenverkleidung im Bereich des Befestigungsbereiches 21 mit einer geeigneten Durchschlußöffnung versehen sein. Bevorzugt wird auch das Metallgitter 13 im Bereich der Öffnung 17 eine durchgehende Öffnung aufweisen.

Sowohl die Ummantelung 15 als auch der Schlauch 16 sind mit einer Umfangsriffelung 16 versehen, wodurch die erforderliche Flexibilität des Schlauches 11 und der Kombination aus Metallgitter 13 und Ummantelung 15 erhöht wird.

Die Öffnung 17 soll in Richtung der Mittelachse 18 gesehen 0,8 bis 1,8 cm lang sein.

Die Einführung der in gefaltetem Zustand auf einem Ballonkatheter fixierten, erfindungsgemässen Prothesen-Schlauchverbindung in die Arterie oder Aorta kann über ein Einführungsbesteck mit einer Schleuse von maximal 12 mm Innen-Durchmesser erfolgen, wie das im folgenden anhand der Figuren 5 bis 11 beschrieben wird.

Nach Figur 5 ist das Schleusenrohr 33 durch ein in den Körper eines Patienten eingeführtes Trokar 34 in den Bereich der Arterie 12 vorgeschoben, in die die beschriebene Prothesen-Schlauchverbindung eingebracht werden soll.

Innerhalb des Schleusenrohres 33 ist ein einen geringfügig kleineren Durchmesser aufweisendes Dilatationsrohr 35 angeordnet, in dem sich frei beweglich eine Kanüle 36 befindet, die in Figur 6 separat dargestellt ist und einen sich vom vorderen bis zum hinteren Ende erstreckenden, relativ steifen Führungsdraht 37 aufnimmt, dessen für die Aufnahme in der Arterie 12 bestimmter Endbereich flexibel ausgebildet ist und der nach Fig. 6 aus beiden Enden deutlich vorsteht.

Das zumindest im proximalen Bereich konisch zulaufende, innen hohle, die Punktionskanüle 36 aufnehmende Dilatationsrohr 35 besteht an seiner Spitze aus weichem, schmiegsamen Kunststoff und erweitert sich von der Spitze nach hinten über eine Länge von 10 cm bis 15 cm. Es ragt aus dem Schleusenrohr 33 vorne heraus. Damit ist gewährleistet, daß bei Schaffung der Öffnung in der Hauptschlagader 12 diese nur langsam aufgedehnt wird und somit größere Einrisse, aus denen es bluten könnte, vermieden werden.

Erfindungsgemäß ist die Kanüle 36 in ihrem proximalen Endbereich gemäß der Darstellung von Figur 7 geformt, d.h. sie weist dort eine relativ scharfe Spitze 39 auf und ist so gekrümmt, daß ihre Austrittsöffnung 40 sich an einer Seite der Kanüle 36 befindet. Dort tritt auch das in die Arterie 12 einzuführende flexible Ende des Drahtes 37 aus, der vom distalen Ende der Kanüle 36 eingeführt wird. Auf diese Weise wird das Umlenken des durch die Kanüle 36 eingeschobenen Drahtes 37 in Richtung der Achse der Arterie 12 begünstigt.

Bei in der Position von Figur 5 nahe der Arterie 12 befindlichem Schleusenrohr 33 wird zunächst durch geeignete Manipulation die Kanüle 36 aus der proximalen Öffnung 35" des Dilatationsrohres 35 ausgefahren und in die Wand der Arterie 12 eingestochen, so daß dort eine Anfangsöffnung 41 punktiert wird (Figur 7).

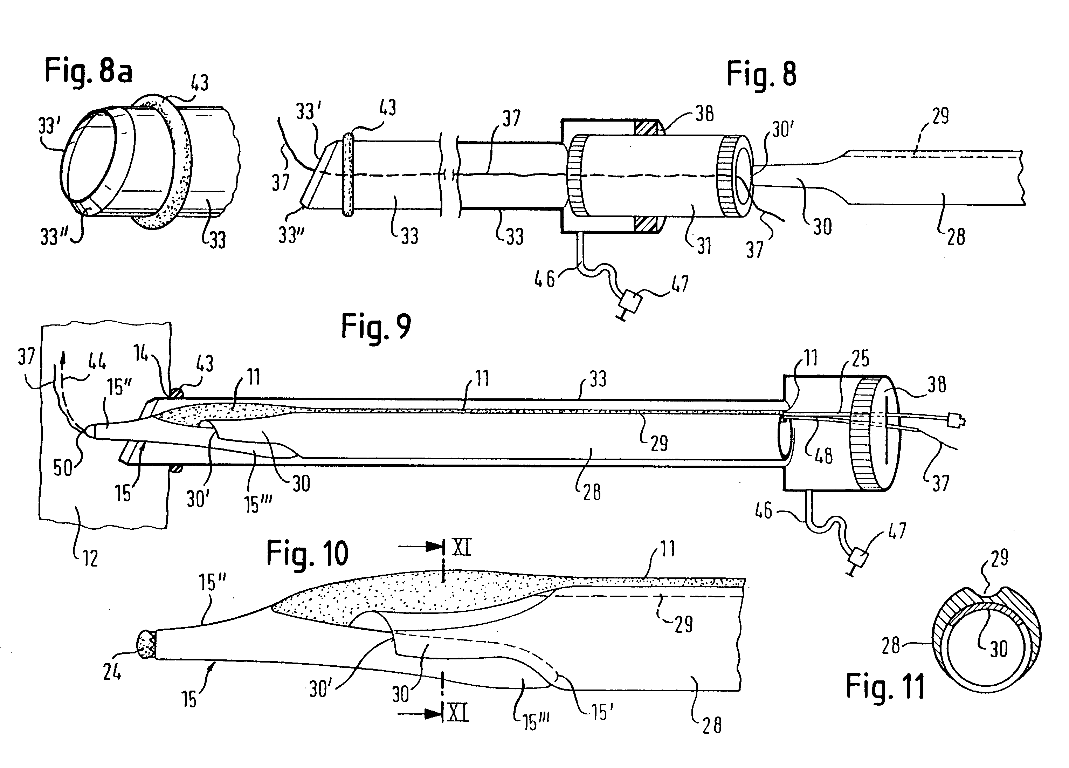

Anschließend wird dann das die Kanüle 36 gemäß Figur 5 umgebende Dilatationsrohr 35 mit seiner sich zur Arterie 12 hin verjüngenden Spitze 35' in die Anfangsöffnung 41 eingeschoben, wodurch gemäß Figur 5 eine erweiterte Öffnung 41' in gewünschter Weise und Größe geschaffen wird. Gleichzeitig wird die Kanüle 36 bei in der Arterie 12 verbleibendem Ende des Drahtes 37 zurückgezogen. Dann wird das Schleusenrohr 33 selbst gemäß Figur 9 in die vorbereitete Öffnung 41' der Arterie 12 eingeschoben, wodurch die endgültige Öffnung 14 in der Arterie 12 entsteht.

Das proximale Ende 33' des Schleusenrohres 33 ist nach den Figuren 5 und 8, 8a etwas zur Längsachse des Schleusenrohres 33 abgeschrägt und weist rundum eine Einführungsschräge 33" auf, hinter der sich ein rundum laufender Dichtungsflansch 43 befindet, der sich bei in die Arterie 12 eingeschobenem Schleusenrohr an die Arterie 12 dichtend anlegt (Figur 9).

Nachdem das Schleusenrohr 33 sich in der Öffnung 14 der Arterie 12 befindet, werden die Kanüle 36 und das Dilatationsrohr 35 aus dem Schleusenrohr 33 herausgezogen, welches an seinem distalen Ende ein Ventil 38 aufweist, das durch flexibles und mit Durchlaßschlitzen versehenes Material gebildet wird, so daß die verschiedenen beschriebenen Bauelemente weitgehend druck- und blutdicht in das Schleusenrohr 33 eingeführt werden können.

Nunmehr wird eine Einführungshülse 31 gemäß Figur 8 von hinten durch das Ventil 38 des Schleusenrohres 33 eingeschoben, um einen axialen Durchlaß für die Einführung eines erfindungsgemäßen Katheters 28 zu schaffen, dessen Ausbildung im einzelnen anhand der Figuren 9, 10, 11 beschrieben wird.

Über den größeren Teil seiner Länge ist der Katheter 28 als kreiszylindrisches Rohr ausgebildet, in dessen Außenumfang sich eine Axialnut 29 befindet, während am vorderen Ende ein rinnenförmiger, schnabelartiger Vorsprung 30 angeformt ist, der teilkreiszylindrisch ausgebildet ist und stetig in das eigentliche Katheterrohr 28 übergeht. Der Vorsprung 30 weist einen etwas geringeren Radius als das Katheterrohr 28 auf und ist konzentrisch mit dem Katheterrohr 28.

Während in den Figuren 8 und 11 das Katheterrohr 28 ohne daran angeordnete Prothesen-Schlauchverbindung gezeigt ist, geben die Figuren 9 und 10 das Katheterrohr 28 mit erfindungsgemäß daran angeordneter Prothesen-Schlauchverbindung wieder.

Nach den Figuren 9 und 10 ist der Verbindungsschlauch 11 der erfindungsgemäßen Prothesen-Schlauchverbindung an der radialen Außenseite des Vorsprungs 30 entlang in die Axialnut 29 am Außenumfang des Katheterrohres 28 eingelegt. Innerhalb des Verbindungsschlauches 11 befindet sich der zum Ballon 24 führende Druckgas-Zuleitungsschlauch 25; er ragt nach Figur 9 hinten aus dem Verbindungsschlauch 11 durch das Ventil 38 nach außen, um dort in nicht dargestellter Weise an eine Druckgasquelle angeschlossen zu werden.

Der durch das Metallgitter 13 mit der Ummantelung 15 gebildete T-Querbalken am proximalen Ende des Verbindungsschlauches 11 ist in der aus den Figuren 9 und 10 ersichtlichen Weise relativ zum Verbindungsschlauch 11 so abgebogen, daß er sich zumindest im wesentlichen in Richtung der Achse des Katheterrohres 28 und fast parallel zum Verbindungsschlauch 11 erstreckt.

Damit der T-Querbalken in dieser federnd an den Verbindungsschlauch 11 weitgehend angelegten Position verbleibt, ist das distale Ende 15' des hinteren Teils 15"' des T-Querbalkens geringfügig in das Katheterrohr 28 eingeführt, wie das in Figur 10 durch eine gestrichelte Linie angedeutet ist. Der Zwickel zwischen dem Teil 15''' und dem Verbindungsschlauch 11 befindet sich an der Stirnseite 30' des Vorsprungs 30. Der Ballon 24 soll an beiden Enden etwas über das Metallgitter 13 vorstehen, um die Verletzungsgefahr beim Einführen zu reduzieren und den im folgenden beschriebenen Lösungseffekt zu begünstigen. Der vordere Teil 15" des T-Querbalkens steht über die Stirnseite 30' des Vorsprungs 30 nach vorn vor, wie das insbesondere aus Figur 10 ersichtlich ist.

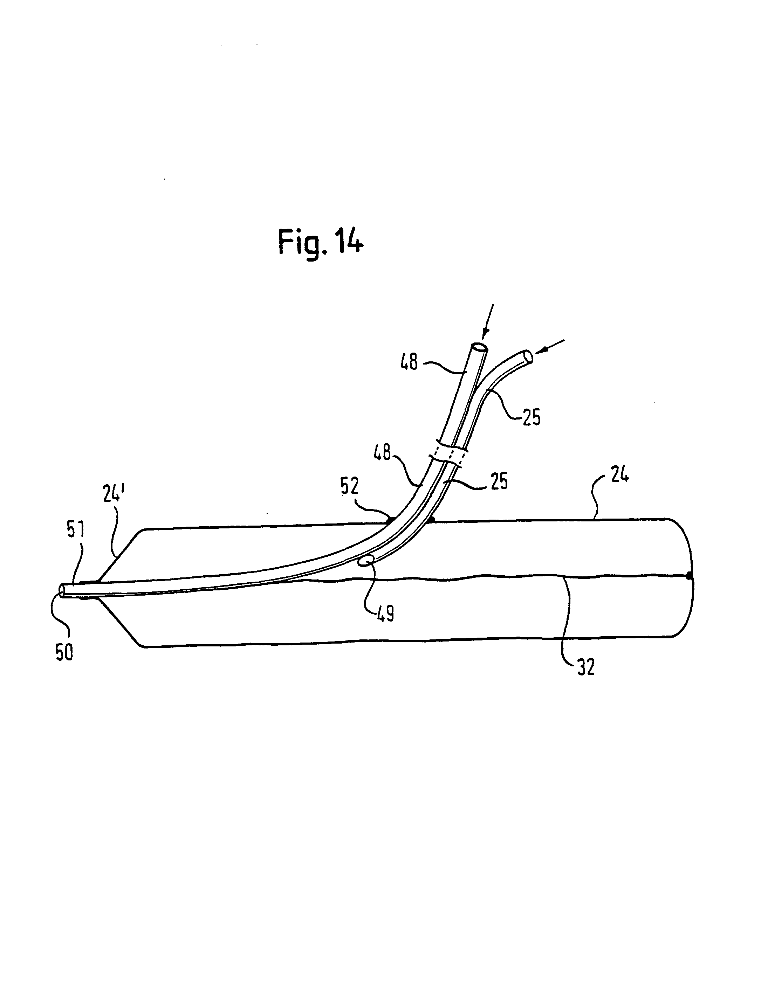

Figur 14 zeigt, daß der Druckgas-Zuleitungsschlauch 25 über eine Klebestelle 52 in das Innere des Ballons 24 eingeführt ist und dort eine Gas-Auslaßöffnung 49 aufweist. Parallel zum Zuleitungsschlauch 25 verläuft ein schlauchförmiger Führungskanal 48, der ebenfalls im Bereich der Klebestelle 52, die sich im Befestigungsbereich 21 (Figur 1) befindet, in das Innere des Ballons 24 eintritt und dort in Richtung der Achse des Ballons 24 abgebogen ist. An der Einführungs-Stirnseite 24' tritt der Führungskanal 48 in Form eines Kanalendstückes 51 aus dem Ballon 24 aus und bildet dort eine Draht-Einführungsöffnung 50. Der Führungskanal 48 ist für die Aufnahme des Drahtes 37 (Figuren 5, 7) bestimmt.

Die beiden bis zu einem nicht dargestellten Anschlußstück parallel und eng aneinander verlaufenden Schläuche 25, 48 können auch konzentrisch zueinander angeordnet sein, derart, daß der Führungskanal 48 zentral und der Zuleitungsschlauch 25 konzentrisch um ihn herum angeordnet ist. Der Zuleitungsschlauch 25 und der Führungskanal 48 sind zweckmäßig wie ein Doppellumen-Katheter ausgebildet. Auf diese Weise bilden der Zuleitungsschlauch 25 und der Führungskanal 48 ein einheitliches Gebilde.

Aus Gründen einer übersichtlichen Darstellung ist der Ballon 24 in Figur 14 im aufgeblasenen Zustand gezeigt.

Bevor der Katheter 28 durch das Ventil 38 hindurch in das Schleusenrohr 33 eingeführt wird, wird der nach Figur 8 aus der Einführungshülse 31 hinten herausragende Draht 37 in die Draht-Einführungsöffnung 50 (Figur 14) eingeführt. Beim Einschieben des Katheters 28 durch die Einführungshülse 31 in das Schleusenrohr 33 verschiebt sich der Draht 37 innerhalb des Führungskanals 48.

Wenn der Katheter 28 mit der so angeordneten Prothesen-Schlauchverbindung durch die Einführungshülse 31 (Figur 8) in das Schleusenrohr 33 eingeschoben wird, verbleibt die Prothesen-Schlauchverbindung so lange in diesem länglichen Zustand, bis nach Figur 9 der proximale Teil 15" des T-Querbalkens in die Arterie 12 eingetreten ist. Hierbei wird der vordere Teil 15" (Figur 9) des T-Querbalkens durch den in die Draht-Einführungsöffnung 50 (Figur 14) eingeführten Draht 37, der sich bis in die Arterie 12 erstreckt, geführt. Der Draht 37 verläuft hierbei etwa so, wie das in Figur 9 angedeutet ist. Nunmehr wird über den Zuleitungsschlauch 25 etwas Druck in den zusammengefalteten Ballon 24 gegeben, der sich gemäß Figur 3 von Anfang an innerhalb des Metallgitters 13 befindet und über den durch den Verbindungsschlauch 11 verlegten Zuleitungsschlauch 25 an eine nicht gezeigte Druckquelle angeschlossen ist.

Durch geringfügiges Aufblasen des Ballons 24 wird erreicht, daß das distale Ende 15' des T-Querbalkens (Figur 10), an dem auch der Ballon 24 etwas vorsteht, aus dem Katheterrohr 28 herausspringt, worauf der T-Querbalken aufgrund der Elastizität der Verbindung zwischen dem Verbindungsschlauch 11 und der Ummantelung 15 versucht, wieder seine normale Winkellage relativ zum Verbindungsschlauch 11 einzunehmen. Das Zurückkehren des T-Querbalkens in seine normale Position relativ zum Verbindungsschlauch 11 kann dadurch unterstützt werden, daß der Katheter beim Einführen in die Arterie etwas zurückgezogen wird. Wird jetzt der Katheter 28 gemäß Figur 9 weiter in die Arterie eingeschoben, so bewegt sich der T-Querbalken geführt vom sich ausreichend weit in die Arterie 12 erstreckenden Draht 37 in Richtung der gestrichelten Linie 44, d.h. in Richtung der Achse der Arterie 12, wobei sowohl das noch gefaltete Metallgitter 13 mit Ummantelung 15 als auch der flexible Vorsprung 30 eine entsprechend der gestrichelten Linie 44 gekrümmte Form annehmen können. Der durch das geringfügige Aufblasen aus dem proximalen Ende vorstehende Endbereich des Ballons 24 reduziert die Verletzungsgefahr.

Sobald das distale Ende 15' des T-Querbalkens (Figur 10) bis in die Arterie 12 gelangt ist, befindet sich der T-Querbalken der Prothesen-Schlauchverbindung in einer parallel zur Arterie 12 verlaufenden Position. Durch anschließendes Zurückziehen des Verbindungsschlauches 11 gelangt der T-Querbalken schließlich in seine endgültige Lage, wie sie beispielsweise in den Figuren 1 und 4 wiedergegeben ist.

Nunmehr kann das Katheterrohr 28 entfernt werden, und der Druck im Ballon 24 wird über den Zuleitungsschlauch 25 auf beispielsweise 10 at erhöht, worauf der Ballon 24 das Metallgitter 13 radial dehnt und die Ummantelung 15 fest an die Innenwand der Arterie 12 preßt.

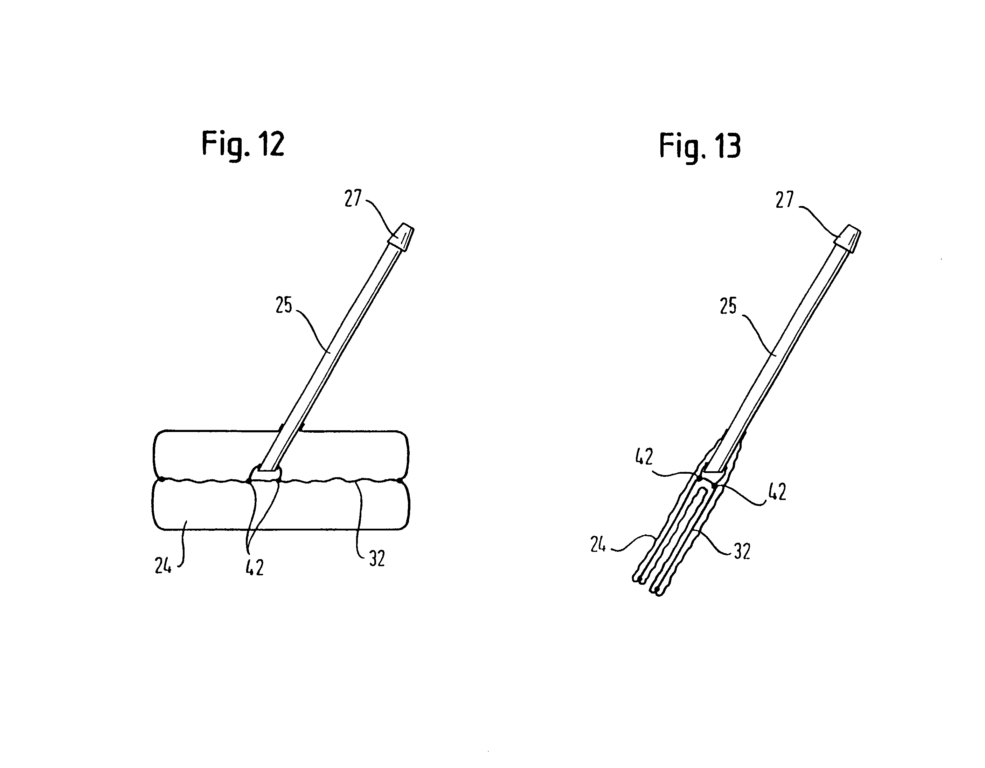

Nachdem die Prothesen-Schlauchverbindung auf diese Weise in der Arterie 12 fixiert ist, wird der Ballon 24 über den Zuleitungsschlauch 25 entlüftet, und der Zuleitungsschlauch 25 mit dem entlüfteten Ballon 24 kann durch den Verbindungsschlauch 11 herausgezogen werden, wobei er die Form nach Figur 13 annimmt.

Nachdem auch das Schleusenrohr 33 entfernt wurde, ist die Prothesen-Schlauchverbindung in der gewünschten Weise blutdicht in der Arterie 12 fixiert und gebrauchsfähig.

Nach Figur 12 kann zur Stabilisierung innerhalb des Ballons 24 eine sich entlang dessen Achse erstreckende Seele 32 angeordnet sein, bis zu der der Druckschlauch 25 reicht, um dort bei 42 an der Seele befestigt zu werden. Die Seele 32 kann auch bei dem Ausführungsbeispiel nach Figur 14 vorgesehen und teilweise durch den axialen Bereich des Führungskanals 48 gebildet sein. Auf diese Weise wird insbesondere das Herausziehen des entlüfteten Ballons 24 gemäß Figur 13 begünstigt und ein zu starkes Ausbeulen des Ballons 24 an seinen Stirnseiten vermieden.

Das Implantationssystem wird also über einen liegenden Trokar 34 (Figur 5) in die Bauchhöhle eingeführt. Bei endoskopischen Operationen ist es üblich, ein Arbeitstrokar 34 in die Bauchdecke des Patienten einzustechen. Das Arbeitstrokar 34 ist mit einem Ventil ausgestattet, so daß man mehrere Instrumente abwechselnd einbringen kann, ohne daß das hergestellte Pneumoperitoneum zusammenfällt.

Durch das Trokar 34 werden das Schleusenrohr 33 mit die Kanüle 36 und den innenliegenden Draht 37 enthaltenden Dilatationsrohr 35 in der in Figur 5 angedeuteten Weise so weit eingeschoben, daß das Dilatationsrohr 35 mit seiner flexiblen Spitze 35' auf den gewünschten Punkt der Schlagader 12 zu liegen kommt. Dann wird gemäß Figur 7 die Kanüle 36 zur Bildung der Anfangsöffnung 41 in die Arterie 12 eingestochen. Anschließend wird gemäß Figur 5 die Spitze 35' des Dilatationsrohres 35 zur Bildung der etwas größeren Öffnung 41' in die Arterie 12 eingeschoben.

Am Ende des Schleusenrohres 33 befindet sich ein hämoestatisches Ventil 38 in einem erweiterten Endbereich 45. Dieses besteht aus Silikon, welches mehrfach kreuzförmig eingeschnitten ist, um einerseits eine Abdichtung nach außen und andererseits die Einführung von Instrumenten zu gestatten. Somit kann kein Blut austreten, wenn man die im Inneren des Schleusenrohres 33 befindlichen Bauelemente auswechselt.

Nachdem gemäß Figur 7 durch die Kanüle 36 eine Punktion der Aorta 12 und anschließend durch das Dilatationsrohr 35 und das Schleusenrohr 33 die Öffnung 14 gewünschter Größe erzeugt worden ist (Figur 9), wird das Dilatationsrohr 35 mit der darin befindlichen Kanüle 36 entfernt, wobei jedoch der Führungsdraht 37 in der Aorta 12 verbleibt.

Nach Entfernung der Dilatationseinheit 35, 36 wird das Schleusenrohr 33 mit Heparin-NaCl-Lösung gespült, wozu am distalen Ende des Schleusenrohres 33 ein Schlauchanschluß 46 mit Drei-Wege-Ventil 47 vorgesehen ist (Figuren 5, 8, 9).

Aufgrund der speziellen Ausbildung der Kanüle 36 nach Figur 7 ist gewährleistet, daß der Draht 37 nur in cranieller Richtung in die Arterie 12 eingeführt werden kann. Nachdem der Draht 37 eingebracht wurde, kann er als Führung für das Dilatationsrohr 35 dienen, das die punktierte Anfangsöffnung 41 in der Arterie 12 zumindest annähernd auf das gewünschte Maß 41' (Figur 5) erweitert.

Der Draht 37 wird soweit in die Aorta 12 eingeführt, daß er beim Zurückziehen der Kanüle 36 nicht aus der Arterie 12 herausgleitet.

Das Einführen des Dilatationsrohres 35 in die punktierte Öffnung 41 der Arterie 12 ist deshalb notwendig, damit keine Blutung auftritt. Die Kanüle 36 besitzt einen größeren Durchmesser als der Führungsdraht 37, so daß beim Entfernen der Kanüle 36 aus der Anfangsöffnung 41 eine Blutung resultieren könnte, wenn nicht zuvor die Spitze 35' des Dilatationsrohres 35 in die Anfangsöffnung 41 eingeschoben wird.

Der Dichtungsflansch 43 im proximalen Endbereich des Schleusenrohres 33 besteht vorzugsweise aus Silikon. Erst wenn der Dichtungsflansch 43 an der Wand der Arterie 12 anliegt, d.h. das distale Ende 33' des Schleusenrohres 33 in die Arterie 12 zur Schaffung der Öffnung 14 in die Arterie 12 eingedrungen ist, wird das Dilatationsrohr 35 entfernt.

Wenn das Dilatationsrohr 35 aus dem Schleusenrohr 33 herausgezogen wird, füllt sich die Schleuse mit Blut. Über das Drei-Wege-Ventil 47 und den Schlauchanschluß 46 wird das System dann mit NaCl-Lösung sowie Heparin 5000 IE-100 ml gespült. Das Ventil 38 am Ende des Schleusenrohres 33 ist hierbei dicht. Ein Eindringen von Luft oder eine Blutung kann nicht erfolgen.

Die Einführhülse 31 (Figur 8) dient zum Durchschieben des die Prothesen-Schlauchverbindung enthaltenden Katheters 28 (Stent-Graft-Systems). Da das hämoestatische Ventil 38 relativ straff ist, könnte bei direkter Einführung der Prothese ein Schaden an derselben entstehen. Um dies zu verhindern, wird mit der Einführhülse 31 das Ventil 38 offengehalten, um so den Katheter 28 mit der Prothesen-Schlauchverbindung gefahrlos in das Schleusenrohr 33 einbringen zu können. Um eine Blutung zu vermeiden, soll in die Einführhülse 31 schon vor dem Einbringen in das Schleusenrohr 33 der vordere Teil des Katheters 28 eingeschoben sein. Der Durchmesser des Katheters 28 sollte dem Innendurchmesser der Einführungshülse 31 entsprechen, um das Durchtreten von Blut nach hinten zu vermeiden.

Der Katheter 28 wird dann durch Einführen des distalen Endes des Drahtes 37 in die Draht-Einführungsöffnung 50 (Figur 9, 14) über den noch im Schleusenrohr 33 liegenden Führungsdraht 37 (Figur 8) geschoben, bis das vordere Ende des Katheters 28 gemäß Figur 9 in die Arterie 12 eingetreten und der T-Querbalken 15 in der Arterie 12 positioniert ist.

Der Draht 37 erfüllt neben der Lage und Positionierung des T-Querbalkens 15 die Aufgabe der Sicherung des Gesamtsystems, d.h. er dient als Leitlinie aller einzubringen Teile. Erst anschließend wird der Führungsdraht 37 herausgezogen und schließlich der Ballon 24 über den Zuleitungsschlauch 25 geringfügig aufgeblasen, damit der T-Querbalken 15", 15"' der Prothesen-Schlauchverbindung frei wird, wie das oben beschrieben wurde. Nachdem der T-Querbalken der Prothesen-Schlauchverbindung frei und vollständig in der Arterie 12 angeordnet ist, kann durch Zurückziehen des Einführbestecks der T-Querbalken in die richtige Position in der Arterie 12 gebracht werden. Hierbei befindet sich das Schleusenrohr 33 noch immer in der aus Figur 9 ersichtlichen Position innerhalb der Arterie 12.

Eine Umlenkung oder wesentliche Veränderung des Trokars 34, welches nur in Figur 5 dargestellt ist, ist nicht erforderlich. Nachdem der Ballon 24 vollständig ausgedehnt wurde, sich also der T-Querbalken der erfindungsgemäßen Prothesen-Schlauchverbindung in der richtigen Position in der Arterie 12 befindet, wird das Schleusenrohr 33 durch den in der Arterie 12 mittels des Ballons 24 erzeugten Druckes aus der Arterie 12 herausgeschoben. Dabei wird mit Drücken um 10 at gearbeitet. Sollte das Schleusenrohr 33 hierbei nur noch wenige mm in der Wandung der Arterie 12 stecken, kann es in dieser Lage verbleiben. Wenn der Ballon 24 dann im entlüfteten Zustand in den Verbindungsschlauch 11 gezogen wird, kann er durch Einspeisen von Druckgas erneut etwas aufgedehnt werden, wodurch das Schleusenrohr 33 automatisch rund 1 bis 2 cm von der Arterie 12 weggeschoben wird.

Der liegende Führungsdraht 37, der über die Punktionskanüle 36 in die Arterie 12 eingebracht wird, dient zur Richtungsdefinition und Lagekontrolle des Dilatationsrohres 35, des Schleusenrohres 33 sowie des Katheters 28. Bevorzugt ist der Draht 37 steif und weist eine 50 mm bis 100 mm lange, flexible Spitze auf, die zum Einführen in die Arterie bestimmt ist.

Der Vorsprung 30 am proximalen Ende des Katheters 28 soll so gekrümmt und dimensioniert sein, daß er die Ummantelung 15 des Metallgitters 15 um mehr als die Hälfte umfaßt. Da das Material des Vorsprunges 30 weich und nachgiebig ist, kann bei einer Füllmenge von weniger als 10 ml der T-Querbalken 15", 15''' aus seiner Verankerung im Katheterrohr 28 gelöst werden.

Die Einführungshülse 31 verbleibt auch zur Blutungskontrolle in dem Schleusenrohr 33. Erst wenn der Ballon 24 vollständig aufgedehnt wurde und unter Druck gehalten wird, kann die Einführungshülse 31 entfernt werden. Um Blutungen zu vermeiden, ist die Einführungshülse 31 ihrerseits dicht innerhalb des von ihr geöffneten Ventils 38 anzuordnen.

In den Endbereichen der verschiedenen in das Schleusenrohr 33 einzuführenden Bauelemente (z.B. 36, 35) sind zweckmäßigerweise Markierungen 53 (Figuren 5, 6) vorgesehen, die mit Gegenmarkierungen am Schleusenrohr 33 zusammenarbeiten, um bei den Manipulationen erkennen zu können, wenn das proximale Ende des betreffenden Bauelements seine Arbeitsposition erreicht hat. Besonders wichtig ist dies bei der Kanüle 36 (Figuren 6, 7), damit das richtige Maß des Eindringens der Kanüle 36 in die Arterie 12 problemlos gefunden werden kann.

Die ungefähren Abmessungen der einzelnen Bauelemente des erfindungsgemäßen Einführungsbestecks sind wie folgt:
Innendurchmesser des Katheters 28: 8 bis 15 mm.
Tiefe der Axialnut 29: 2 bis 3 mm.
Länge des Vorsprunges 30: 10 bis 30 mm.
Länge der Einführungshülse 31: 60 mm.
Durchmesser der Einführungshülse 31: 9 bis 16 mm.
Durchmesser des Drahtes 37: 0,1 mm.
Durchmesser des Dilatationsrohres 35: 8 bis 15 mm.

Länge der konischen Spitze 35' des Dilatationsrohres 35: bis 100 mm. Abstand des Endes 33' des Schleusenrohres 33 von dem Dichtungsflansch 43: 0,4 mm bis 0,6 mm.
Innendurchmesser des Schleusenrohres 33: 9 bis 18 mm.
Durchmesser des Ballons 24: 8 mm bis 22 mm.
Länge des Ballons 24: 40 mm.
Expansionsdruck des Ballons 24: 8 at bis 15 at.
Durchmesser des Zuführungsschlauches 25: 3 mm.
Länge des Zuleitungsschlauches (und des Führungskanals 48): 60 cm.

### Bezugszeichenliste

- 11: Prothesen-Verbindungsschlauch
- 12: Arterie
- 13: Metallgitter
- 13': Teil des Metallgitters
- 13": Teil des Metallgitters
- 13"': freies Ende des Metallgitters
- 14: Längsschnitt
- 15: Ummantelung
- 15': distales Ende
- 15": Teil
- 15"': Teil
- 16: Umfangsriffelung
- 17: Öffnung
- 18: Mittelachse
- 19: stromaufwärtiger Teil
- 20: stromabwärtiger Teil
- 21: Befestigungsbereich
- 22: Naht
- 23: Blutstromrichtung
- 24: Ballon
- 24': Stirnseite
- 25: Zuleitungsschlauch
- 26: Ventil
- 27: Anschlußkonus
- 28: Katheter
- 29: Axialnut
- 30: Vorsprung
- 30': Stirnseite
- 31: Einführungshülse
- 32: Seele
- 33: Schleusenrohr
- 33': Ende
- 33": Einführungsschräge
- 34: Trokar
- 35: Dilatationsrohr
- 35': Spitze
- 35": Öffnung
- 36: Kanüle
- 37: Draht
- 38: Ventil
- 39: Spitze
- 40: Austrittsöffnung
- 41: Anfangsöffnung
- 42: Befestigungsstelle
- 43: Dichtungsflansch
- 44: Linie
- 45: Endbereich
- 46: Schlauchanschluß
- 47: Drei-Wege-Ventil
- 48: Führungskanal
- 49: Gas-Auslaßöffnung
- 50: Draht-Einführungsöffnung
- 51: Kanalendstück
- 52: Klebestelle
- 53: Markierung

## Patentansprüche

1. Einführungsbesteck für eine Prothesen-Schlauchverbindung zur Herstellung einer Verbindung zwischen zwei Blutgefäßen, insbesondere Arterien (12) bzw. Arterienbereichen, wobei das Einführungsbesteck eine Prothesen - Schlauchunbindung mit einem flexiblen, blutundurchlässigen Verbindungsschlauch (11), der an wenigstens einem und vorzugsweise nur an einem Ende ein unter einem Winkel zu seiner Längsachse angeordnetes expandierbares, rohrförmiges Metallgitter (13) mit einer blutdichten Innenverkleidung und/oder Ummantelung (15) trägt, welches sich vom betreffenden Ende des Schlauches (11) T-querbalkenartig nach zwei entgegengesetzten Richtungen erstreckt, wobei sich innerhalb des Metallgitters (13) ein durch ein Druckfluid expandierbarer Expansionsballon (24) befindet, der über einen vorzugsweise durch den Verbindungsschlauch (11) geführten Druckfluid-Zuführungsschlauch (25) zwecks Expansion des Metallgitters (13) mit Druckfluid beaufschlagbar ist, sowie einen Katheter aufweist,
wobei
das Metallgitter (13) mit Innenverkleidung und/oder Ummantelung (15) sowie Expansionsballon (24) in relativ zum Verbindungsschlauch (11) verschwenktem Zustand am proximalen Ende des Katheters (28) so gehalten ist, daß dadurch der durch das Metallgitter (13) mit Innenverkleidung und/oder Ummantelung (15) sowie Expansionsballon (24) gebildete T-Querbalken sich zumindest annähernd in Längsrichtung des Katheters (28) erstreckt.

2. Einführungsbesteck nach Anspruch 1,
**dadurch gekennzeichnet,**
**daß** der Katheter (28) als Stange oder Rohr ausgebildet ist.

3. Einführungsbesteck nach Anspruch 1 oder 2,
**dadurch gekennzeichnet,**
**daß** der Verbindungsschlauch (11) mit dem darin verlaufenden Zuführungsschlauch (25) an der Außenseite des Katheters (28) in Längsrichtung desselben angeordnet ist.

4. Einführungsbesteck nach Anspruch 3,
**dadurch gekennzeichnet,**
**daß** in der Außenwand des Katheters (28) eine den Verbindungsschlauch (11) aufnehmende Axialnut (29) vorgesehen ist.

5. Einführungsbesteck nach einem der vorhergehenden Ansprüche,
**dadurch gekennzeichnet,**
**daß** der proximale Endbereich des Katheters (28) einseitig offen ist und der an den Verbindungsschlauch (11) herangeschwenkte Teil (15"') des T-Querbalkens in dieser Öffnung liegt und vorzugsweise mit seinem distalen Ende (15') am Katheter (28) in der zumindest im wesentlichen parallel zur Katheterachse verlaufenden Position lösbar gehalten ist, während der andere, vom Verbindungsschlauch (11) weggeschwenkte Teil (15") des T-Querbalkens nach vorn über das proximale Ende des Katheters (28) vorsteht.

6. Einführungsbesteck nach Anspruch 5,
**dadurch gekennzeichnet,**
**daß** die seitliche Öffnung des proximalen Endbereiches des Katheters (28) durch Vorsehen eines flexiblen schnabelartigen Vorsprunges (30) am proximalen Ende des Katheters (28) realisiert ist, entlang dessen sich der herangeschwenkte Teil (15"') des T-Querbalkens zumindest teilweise erstreckt.

7. Einführungsbesteck nach Anspruch 5 oder 6,
**dadurch gekennzeichnet,**
**daß** der Verbindungsschlauch (11) und der herangeschwenkte Teil (15"') des T-Querbalkens sich auf entgegengesetzten Seiten des Vorsprunges (30) entlang desselben erstrecken.

8. Einführungsbesteck nach einem der Ansprüche 5 bis 7,
**dadurch gekennzeichnet,**
**daß** der Vorsprung (30) rinnenförmig ausgebildet ist und die Rohrform des Katheters (28) teilweise zumindest im wesentlichen fortsetzt, wobei der rinnenförmige Vorsprung (30) vorzugsweise zumindest im wesentlichen konzentrisch zum Katheterrohr (28) angeordnet ist.

9. Einführungsbesteck nach einem der Ansprüche 6 bis 8,
**dadurch gekennzeichnet,**
**daß** der Radius des Vorsprunges (30) etwas geringer als der des Katheterrohrs (28) ist.

10. Einführungsbesteck nach einem der Ansprüche 5 bis 9,
**dadurch gekennzeichnet,**
**daß** der Vorsprung (30) geringfügig kürzer als der in ihm aufgenommene Teil (15''') des T-Querbalkens ist, derart, daß das freie Ende (15') dieses Teils (15"') etwas in das Katheterrohr (18) hineinreicht und der T-Querbalken somit zumindest annähernd parallel zum Katheterrohr (28) gehalten ist.

11. Einführungsbesteck nach Anspruch 10,
**dadurch gekennzeichnet,**
**daß** die Überlappung des von dem Vorsprung (18) aufgenommenen Teils (13") des Metallgitters (13) mit dem Katheterrohr (28) nur so groß ist, daß das durch etwas Druckerzeugung im Zuführungsschlauch (25) bzw. Ballon (24) das Teil (13") aus dem Katheterrohr (28) herausschnappen kann.

12. Einführungsbesteck nach einem der Ansprüche 5 bis 11,
**dadurch gekennzeichnet,**
**daß** der Umfang des flexiblen Vorsprunges (30) so begrenzt und die Flexibilität so groß ist, daß er sich beim Einführen in eine Arterie (12) aus der Einführungsrichtung in die Arterienrichtung abbiegen kann.

13. Einführungsbesteck nach einem der vorhergehenden Ansprüche,
**dadurch gekennzeichnet,**
**daß** ein in eine Öffnung (32) der Arterie (12) dicht einsetzbares Schleusenrohr (33) vorgesehen ist, dessen Innendurchmesser so groß ist, daß das Katheterrohr (28) axial hindurchgeführt werden kann.

14. Einführungsbesteck nach Anspruch 13,
**dadurch gekennzeichnet,**
**daß** das Schleusenrohr (33) innerhalb eines Trokars (34) angeordnet ist, welches von außen in den Körper des Patienten einführbar ist.

15. Einführungsbesteck nach Anspruch 13 oder 14,
**dadurch gekennzeichnet,**
**daß** ein Dilatationsrohr (35) vorgesehen ist, welches statt des Katheters (28) im Schleusenrohr (33) anzuordnen ist und sich im proximalen Endbereich in Richtung der zu punktierenden Arterie (12) verjüngt.

16. Einführungsbesteck nach Anspruch 15,
**dadurch gekennzeichnet,**
**daß** zumindest die Spitze (35') des Dilatationsrohres (35) elastisch und etwas abgebogen ist.

17. Einführungsbesteck nach einem der Ansprüche 15 oder 16,
**dadurch gekennzeichnet,**
**daß** innerhalb des Dilatationsrohres (35) eine Kanüle (36) angeordnet ist, mit der die Arterie (12) eines Patienten punktierbar ist.

18. Einführungsbesteck nach Anspruch 17,
**dadurch gekennzeichnet,**
**daß** die Kanülenöffnung (40) hinter der Spitze (39) seitlich angeordnet und die Kanülenwand gegenüber der Kanülenöffnung (40) konkav gekrümmt ist.

19. Einführungsbesteck nach Anspruch 17 oder 18,
**dadurch gekennzeichnet,**
**daß** innerhalb der Kanüle (36) ein schubsteifer, jedoch zumindest im in die Arterie (12) einzuführenden Endbereich flexibler elastischer Draht (37) vorgesehen ist, der bei in die Arterie (12) des Patienten eingestochener Kanüle (36) durch letztere in die Arterie (12) einführbar ist.

20. Einführungsbesteck nach Anspruch 19,
**dadurch gekennzeichnet,**
**daß** am bzw. im Expansionsballon (24) und Zuleitungsschlauch (25) ein durchgehender Kanal (48) für die Aufnahme des Drahtes (37) vorgesehen ist.

21. Einführungsbesteck nach einem der vorhergehenden Ansprüche,
**dadurch gekennzeichnet,**
**daß** der Expansionsballon (24) eine ihn in Längsrichtung stabilisierende, vorzugsweise flexible Seele (32) aufweist.

22. Einführungsbesteck nach einem der vorhergehenden Ansprüche,
**dadurch gekennzeichnet,**
**daß** der Ballon (24) zumindest am Einführungsende (24'), vorzugsweise jedoch an beiden Enden etwas über das Metallgitter (13) mit Innenverkleidung und/oder Ummantelung (15) vorsteht.

23. Einführungsbesteck nach einem der Ansprüche 13 bis 22,
**dadurch gekennzeichnet,**
**daß** am hinteren Ende des Schleusenrohres (33) ein Ventil (38) vorgesehen ist, durch das hindurch die verschiedenen Instrumentarien (28, 31, 35, 36, 37) zumindest weitgehend druckdicht einführbar sind.

24. Einführungsbesteck nach Anspruch 23,
**dadurch gekennzeichnet,**
**daß** eine Einführungshülse (31) für das Katheterrohr (28) vorgesehen ist, welche durch das Ventil (38) in den hinteren Endbereich des Schleusenrohres (33) blutdicht einführbar ist.

## Claims

1. An instrument for inserting a prosthesis hose connection for the establishing of a connection between two blood vessels, in particular arteries (12), or arterial regions, with the insertion instrument having a prosthesis hose connection as well as a catheter, the prosthesis hose connection having a flexible connecting hose (11) which is impermeable to blood and which carries an expandable tubular metal grid (13) arranged at an angle to its longitudinal axis at at least one end, and preferably only at one end, said metal grid (13) having a blood impermeable inner liner and/or covering (15) and extending from the relevant end of the hose (11) like a cross bar of a T in two opposite directions, with an expansion balloon (24) expandable by a pressurized fluid being disposed within the metal grid (13) and being preferably charged with pressurized fluid via a pressurized fluid delivery hose (25) guided through the connecting hose (11) for the expansion of the metal grid (13), as well as a catheter, wherein the metal grid (13) with the inner liner and/or covering (15) and the expansion balloon (24), is held at the proximal end of the catheter (28) in a state pivoted relative to the connecting hose (11) such that the cross bar of a T formed by the metal grid (13), with the inner liner and/or covering (15) and the expansion balloon (24), thereby extends at least approximately in the longitudinal direction of the catheter (28).

2. An insertion instrument in accordance with claim 1, **characterized in that** the catheter (28) is made as a rod or tube.

3. An insertion instrument in accordance with claim 1 or claim 2, **characterized in that** the connecting hose (11), with the delivery hose (25) arranged therein, is arranged at the outer side of the catheter (28) in the longitudinal direction of the same.

4. An insertion instrument in accordance with claim 3, **characterized in that** an axial groove (29) accepting the connecting hose (11) is provided in the outer wall of the catheter (28).

5. An insertion instrument in accordance with any one of the preceding claims, **characterized in that** the proximal end region of the catheter (28) is open at one side and the part (15"') of the cross bar of a T pivoted toward the connecting hose (11) lies in this opening and is preferably releasably held with its distal end (15') at the catheter (28) in the position extending at least substantially parallel to the catheter axis, while the other part (15") of the cross bar of a T pivoted away from the connecting hose (11) projects forwardly beyond the proximal end of the catheter (28).

6. An insertion instrument in accordance with claim 5, **characterized in that** the side opening of the proximal end region of the catheter (28) is realized by provision of a flexible, lip-shaped projection (30) at the proximal end of the catheter (28), along which the part (15"') of the cross bar of a T pivoted toward it extends at least in part.

7. An insertion instrument in accordance with claim 5 or claim 6, **characterized in that** the connecting hose (11) and the part (15"') of the cross bar of a T pivoted toward it extend on opposite sides of the projection (30) along the same.

8. An insertion instrument in accordance with any one of claims 5 to 7, **characterized in that** the projection (30) is made in channel-like form and the tube shape of the catheter (38) continues at least substantially in part, with the channel-like projection (30) preferably being arranged at least substantially concentrically to the catheter tube (28).

9. An insertion instrument in accordance with any one of claims 6 to 8, **characterized in that** the radius of the projection (30) is slightly smaller than that of the catheter tube (28).

10. An insertion instrument in accordance with any one of claims 5 to 9, **characterized in that** the projection (30) is slightly shorter than the part (15"') of the cross bar of a T received in it such that the free end (15') of this part (15"') reaches somewhat into the catheter tube (18) and the cross bar of a T is thus held at least approximately parallel to the catheter tube (28).

11. An insertion instrument in accordance with claim 10, **characterized in that** the overlapping of the part (13") of the metal grid (13) received by the projection (18) with the catheter tube (28) is only so large that the part (13") can snap out of the catheter tube (28) by some pressure generation in the delivery hose (25) or balloon (24).

12. An insertion instrument in accordance with any one of claims 5 to 11, **characterized in that** the periphery of the flexible projection (30) is so restricted and the flexibility is so large that, on insertion into an artery (12), it can bend out of the insertion direction into the direction of the artery.

13. An insertion instrument in accordance with any one of the preceding claims, **characterized in that** a lock tube (33) is provided which is tightly insertable into an opening (32) of the artery (12) and whose inner diameter is so large that the catheter tube (28) can be axially guided through.

14. An insertion instrument in accordance with claim 13, **characterized in that** the lock tube (33) is arranged inside a trocar (34) which is insertable into the body of the patient from the outside.

15. An insertion instrument in accordance with claim 13 or claim 14, **characterized in that** a dilation tube (35) is provided which is arranged in the lock tube (33) instead of the catheter (28) and tapers in the direction toward the artery (12) to be punctured in the proximal end region.

16. An insertion instrument in accordance with claim 15, **characterized**
**in that** at least the tip (35') of the dilation tube (35) is elastic and somewhat bent.

17. An insertion instrument in accordance with either of claims 15 or 16, **characterized in that** a cannula (36), with which the artery (12) of a patient can be punctured, is arranged inside the dilation tube (35).

18. An insertion instrument in accordance with claim 17, **characterized in that** the cannula opening (40) is arranged to the side behind the tip (39) and the cannula wall is concavely curved with respect to the cannula opening (40).

19. An insertion instrument in accordance with claim 17 or claim 18, **characterized in that** a wire (37) stiff in shear is provided inside the cannula (36) and is, however, flexible and elastic at least in the end region to be inserted into the artery (12) and, with the cannula (36) inserted into the artery (12) of the patient, can be inserted through the former into the artery (12).

20. An insertion instrument in accordance with claim 19, **characterized in that** a throughgoing channel (48) is provided for the reception of the wire (37) at or in the expansion balloon (24) and delivery hose (25).

21. An insertion instrument in accordance with any one of the preceding claims, **characterized in that** the expansion balloon (24) has a core (32), preferably flexible, which stabilizes it in the longitudinal direction.

22. An insertion instrument in accordance with any one of the preceding claims, **characterized in that** the balloon (24) projects, at least at the insertion end (24'), preferably, however, at both ends, somewhat beyond the metal grid (13) with the inner liner and/or covering (15).

23. An insertion instrument in accordance with any of claims 13 to 22, **characterized in that** a valve (38) is provided at the rear end of the lock tube (33) through which the different instruments (28, 31, 35, 36, 37) can be inserted in at least a largely pressure tight manner.

24. An insertion instrument in accordance with claim 23, **characterized**
**in that** an insertion sleeve (31) is provided for the catheter tube (28) and can be inserted in a blood impermeable manner through the valve into the rear end region of the lock tube (33).

## Revendications

1. Instrument d'introduction pour une jonction tubulaire prothétique destinée à établir une liaison entre deux vaisseaux sanguins, en particulier des artères (12) ou des morceaux d'artère, ledit instrument d'introduction comprenant une jonction tubulaire prothétique avec un tuyau de jonction (11) souple imperméable vis-à-vis du sang, qui porte à au moins une extrémité et de préférence à une extrémité seulement un grillage métallique (13) de forme tubulaire capable de se dilater et agencé sous un angle par rapport à son axe longitudinal avec un habillage intérieur et/ou un habillage extérieur (15) étanche vis-à-vis du sang, qui s'étend depuis l'extrémité concernée du tuyau (11) à la manière de la traverse d'un T dans les deux directions opposées, dans lequel à l'intérieur du grillage métallique (13) est prévu un ballon de dilatation (24) capable d'être dilaté au moyen d'un fluide sous pression et qui peut être alimenté avec un fluide sous pression via un tube d'alimentation (25) de fluide sous pression passé de préférence à travers le tuyau de jonction (11) dans le but de faire dilater le grillage métallique (15), et comprenant un cathéter,
dans lequel le grillage métallique (13) avec l'habillage intérieur et/ou l'habillage extérieur (15) ainsi que le ballon de dilatation (24) est maintenu à l'extrémité proximale du cathéter (28) dans un état pivoté par rapport au tuyau de jonction (11) de telle manière que la traverse en T formée par le grillage métallique (13) avec l'habillage intérieur et/ou l'habillage extérieur (15) ainsi que le ballon de dilatation (24) s'étend ainsi au moins approximativement en direction longitudinale du cathéter (28).

2. Instrument d'introduction selon la revendication 1, **caractérisé en ce que** le cathéter (28) est réalisé sous forme d'une tige ou d'un tube.

3. Instrument d'introduction selon l'une ou l'autre des revendications 1 et 2, **caractérisé en ce que** le tuyau de jonction (11) avec le tube d'alimentation (25) disposé à l'intérieur est agencé au niveau de la face extérieure du cathéter (28) dans la direction longitudinale de celui-ci.

4. Instrument d'introduction selon la revendication 3, **caractérisé en ce qu'**il est prévu une gorge axiale (29) dans la paroi extérieure du cathéter (28), qui reçoit le tuyau de jonction (11).

5. Instrument d'introduction selon l'une des revendications précédentes, **caractérisé en ce que** la zone terminale proximale du cathéter (28) est ouverte d'un côté, et **en ce que** la partie (15"') de la traverse du T pivotée en rapprochement du tuyau de jonction (11) est disposée dans cette ouverture, et de préférence maintenue par son extrémité distale (15') sur le cathéter (28) de manière détachable dans la position qui s'étend au moins sensiblement parallèlement à l'axe du cathéter, tandis que l'autre partie (15") de la traverse du T pivotée en éloignement du tuyau de jonction (11) dépasse vers l'avant au-delà de l'extrémité proximale du cathéter (28).

6. Instrument d'introduction selon la revendication 5, **caractérisé en ce que** l'ouverture latérale de la zone terminale proximale du cathéter (28) est réalisée en prévoyant une saillie (30) souple en forme de bec à l'extrémité proximale du cathéter (28), le long de laquelle s'étend au moins partiellement la partie (15"') de la traverse du T pivotée en rapprochement.

7. Instrument d'introduction selon l'une ou l'autre des revendications 5 et 6, **caractérisé en ce que** le tuyau de jonction (11) et la partie (15"') de la traverse du T pivotée en rapprochement s'étendent sur des côtés opposés de la saillie (30) et le long de celle-ci.

8. Instrument d'introduction selon l'une des revendications 5 à 7, **caractérisé en ce que** la saillie (30) est réalisée sous la forme d'une nervure et prolonge au moins sensiblement partiellement la forme tubulaire du cathéter (28), la saillie (30) en forme de nervure étant de préférence agencée au moins sensiblement concentrique au tube de cathéter (28).

9. Instrument d'introduction selon l'une des revendications 6 à 8, **caractérisé en ce que** le rayon de la saillie (30) est quelque peu inférieur à celui du tube de cathéter (28).

10. Instrument d'introduction selon l'une des revendications 5 à 9, **caractérisé en ce que** la saillie (30) est légèrement plus courte que la partie (15''') de la traverse du T qu'elle reçoit, de telle façon que l'extrémité libre (15') de cette partie (15"') pénètre légèrement dans le tube de cathéter (18), et la traverse du T est ainsi maintenue au moins approximativement parallèle au tube de cathéter (28).

11. Instrument d'introduction selon la revendication 10, **caractérisé en ce que** le chevauchement de la partie (13") du grillage métallique (13) reçu par la saillie (18) et du tube de cathéter (28) a une taille juste de suffisante pour qu'en appliquant une légère pression dans le tube d'alimentation (25) ou dans le ballon (24) ladite partie (13") est capable de s'échapper hors du tube de cathéter (28).

12. Instrument d'introduction selon l'une des revendications 5 à 11, **caractérisé en ce que** la périphérie de la saillie souple (30) est délimitée de telle façon, et **en ce que** la flexibilité est si importante que lors de l'introduction dans une artère (12) la saillie est capable de fléchir depuis une direction d'introduction jusque dans la direction de l'artère.

13. Instrument d'introduction selon l'une des revendications précédentes, **caractérisé en ce qu'**il est prévu un tube-sas (33) susceptible d'être mis en place de façon étanche dans une ouverture (32) de l'artère (12), et dont le diamètre intérieur est si important que le tube de cathéter (28) peut le traverser axialement.

14. Instrument d'introduction selon la revendication 13, **caractérisé en ce que** le tube-sas (33) est agencé à l'intérieur d'un trocart (34) susceptible d'être introduit depuis l'extérieur dans le corps du patient.

15. Instrument d'introduction selon l'une ou l'autre des revendications 13 et 14, **caractérisé en ce qu'**il est prévu un tube de dilatation (35) destiné à être agencé dans le tube-sas (33) au lieu du cathéter (28), et qui va en se rétrécissant dans la zone terminale proximale en direction de l'artère (12) à perforer.

16. Instrument d'introduction selon la revendication 15, **caractérisé en ce que** la pointe (35') au moins du tube de dilatation (35) est élastique et légèrement cintrée.

17. Instrument d'introduction selon l'une ou l'autre des revendications 15 et 16, **caractérisé en ce qu'**une canule (36) est agencée à l'intérieur du tube de dilatation (35), au moyen de laquelle l'artère (12) d'un patient peut être perforée.

18. Instrument d'introduction selon la revendication 17, **caractérisé en ce que** l'ouverture de canule (40) est agencée latéralement derrière la pointe (39) et **en ce que** la paroi de canule est incurvée de façon concave par rapport à l'ouverture de canule (40).

19. Instrument d'introduction selon l'une ou l'autre des revendications 17 et 18, **caractérisé en ce qu'**à l'intérieur de la canule (16) est prévu un fil (37) rigide vis-à-vis de la poussée, mais flexible et élastique au moins dans la zone terminale destinée à être introduite dans l'artère (12), ledit fil étant introduit dans l'artère (12) en traversant la canule (36) enfoncée dans l'artère (12) du patient.

20. Instrument d'introduction selon la revendication 19, **caractérisé en ce qu'**un canal traversant (48) pour la réception du fil (37) est prévu sur ou dans le ballon de dilatation (24) et le tube d'alimentation (25).

21. Instrument d'introduction selon l'une des revendications précédentes, **caractérisé en ce que** le ballon de dilatation (24) comporte une âme (32) qui le stabilise en direction longitudinale, de préférence une âme flexible.

22. Instrument d'introduction selon l'une des revendications précédentes, **caractérisé en ce que** le ballon (24) dépasse. légèrement au-delà du grillage métallique (13) avec l'habillage intérieur et/ou l'habillage extérieur (15) au moins à l'extrémité d'introduction (24') et de préférence aux deux extrémités.

23. Instrument d'introduction selon l'une des revendications 13 à 22, **caractérisé à ce qu'**à l'extrémité postérieure du tube-sas (33) est prévue une valve (33) à travers laquelle peuvent être introduits les divers instruments (28, 31, 35, 36, 37) au moins largement de façon étanche vis-à-vis de la pression.

24. Instrument d'introduction selon la revendication 23, **caractérisé en ce qu'**il est prévu une douille d'introduction (31) pour le tube de cathéter (28), laquelle peut être introduite de façon étanche vis-à-vis du sang à travers la valve (39) jusque dans la zone terminale postérieure du tube-sas (33).
